# EUROPEAN PATENT SPECIFICATION

(11) **EP 1 675 638 B1**
(45) Date of publication and mention of the grant of the patent: **04.04.2012**
(21) Application number: 04774912.2
(22) Date of filing: 01.09.2004
(51) Int. Cl.: A61M 16/00, A61M 16/20

(54) **Manually operated apparatus, and ballon unit and valve housing for a manually operated respiration apparatus**
Von Hand betriebenes Gerät und Ballon und Ventilgehäuse für ein manuell betriebenes Atemgerät
Appareil respiratoire à actionnement manuel et unite de ballon et boitier de clapet destinés a un appareil respiratoire à actionnement manuel

(30) Priority: 01.09.2003 NL 1024206
(43) Date of publication of application: 05.07.2006
(73) Proprietor: Emergency Pulmonary Care B.V., 2251 AP Voorschoten (NL)
(72) Inventor: LUGTIGHEID, Gerardus, Wilhelmus, NL-3206 BW Spijkenisse (NL)
(74) Representative: van Loon, C.J.J.
(86) International application number: PCT/NL2004/000606
(87) International publication number: WO 2005/021074

(56) References cited:
- EP-A1- 1 382 364
- WO-A-01/66175
- FR-A- 1 337 050
- FR-A- 1 516 113
- US-A- 5 301 667

## Description

The invention relates to a manually operated respiration apparatus, and to a balloon unit and to a valve housing for a manually operated respiration apparatus.

Manually operated respiration apparatuses are known from practice and usually comprise a respiration housing, provided with a respiration channel for connection with a patient to whom artificial respiration is to be applied, which respiration channel is connected via a valve system in the respiration housing with an inflow port which is connected to a balloon unit for supplying air to the patient via the respiration channel, and which is further connected with an outflow port opening into the environment for evacuating air exhaled by the patient via the respiration channel to the environment. Here, the balloon unit comprises a resilient bellows provided with an inlet opening and an outlet opening, with a non-return valve being included in the inlet opening and with the outlet opening being connected to the inflow port of the respiration housing, all this such that, upon squeezing the bellows from a starting position, air enclosed by the bellows exits via the outlet opening and that, upon releasing the bellows, it rebounds to the starting position by sucking in environmental air via the inlet opening.

Such a manually operated respiration apparatus is known to a skilled person by the name of resuscitator and is used for manually applying artificial respiration to persons or animals, for instance during reanimation see e.g. FR 1516113. Such a respiration apparatus has a simple construction and can easily be transported. Further, the apparatus has a high reliability, while a minimum of maintenance is required. A drawback of the known respiration apparatus is that, thereby, the volume of the lungs of the patient can be increased too much, so that the lungs stretch too far. Such an overstretching of the lung leads to damage and dying off of lung cells, so that the oxygen transfer capacity of the lungs decreases. Although, after a few weeks, the lung cells have regenerated again and the oxygen transfer capacity is restored again, precisely the temporary decrease of the oxygen transfer capacity of the lungs can inflict great damage to the patient and even cause death of the patient.

In order to obviate this drawback, its has already been proposed to include an overpressure protection in the respiration housing to limit the pressure of the air supplied via the respiration channel at a predetermined value.

However, it has been found in practice that, despite this overpressure protection, overstretching of the lung is still caused during artificial respiration.

The invention contemplates a manually operated respiration apparatus of the type referred to in the introduction, by means of which these drawbacks can be prevented while preserving the advantages.

For this purpose, the respiration apparatus according to the invention is characterized in that the balloon unit is further provided with an overpressure protection for evacuating air from the bellows to the environment when a predetermined pressure of the air in the bellows is exceeded.

It has been found that, in this manner, lung damage can be prevented in practice. The invention resides in the insight that, during the bustle of the reanimation and/or in case of use by less expert or untrained staff, the bellows can sometimes be squeezed too forcefully, so that a great pressure wave may be created which can insufficiently rapidly be evacuated via the overpressure protection included in the respiration housing, so that a considerable part of the pressure wave is still able to propagate via the respiration housing into the lungs of the patient. Particularly with a diseased or damaged lung in which the active volume is limited, this may result in severe damage. By providing the balloon unit with an overpressure protection, a rapid reduction of the pressure wave can be made possible, so that the pressure wave is prevented from reaching the lungs of the patient via the respiration housing and the respiration channel.

The balloon unit, particularly the bellows, is a suitable mounting place for such an overpressure protection, since, on or to the balloon, relatively much space is available for a relatively large outflow area of the overpressure protection, so that the flow resistance of the overpressure protection can be relatively low. Here, the overpressure protection may, for instance, be provided on the body of the bellows, but may also be provided in or to the inlet or outlet opening thereof.

By including an overpressure protection in the inlet opening, it is thus achieved that the overpressure protection can have a relatively large outflow area. Also, by inclusion in the inlet opening, it is achieved that the balloon unit of an already existing respiration apparatus - which is optionally provided with an overpressure protection in the respiration housing - can be equipped with a reliable overpressure protection in a simple manner. Further, this place is the most comfortable both for the operator of the balloon and for the patient.

In an advantageous embodiment, in the inlet opening of the bellows, a substantially cylindrical valve housing is included in which the non-return valve and the overpressure protection are accommodated. The non-return valve and the overpressure protection are preferably integrated, but may also form separate parts. The overpressure protection may, for instance, be designed as a spring-actuated valve. It is noted that the non-return valve and the overpressure protection may be integrated into one part, but that they may also be separate parts.

In a further advantageous embodiment, the cylindrical valve housing is, on an end face, provided with one or more suction openings and, on a part of the cylinder jacket located near the end face, provided with one or more blow-off openings. In this manner, it is achieved that, on its end face, the valve housing can be coupled with, for instance, an oxygen bag, while the blow-off openings remain clear.

In an advantageous manner, the cylindrical valve housing may be provided with a clamp for airtight clamping of the circumferential edge of the inlet opening.

The invention also relates to a method for manually applying artificial respiration to a patient.

Further advantageous embodiments of the invention are shown in the subclaims.

The invention will be further elucidated on the basis of exemplary embodiments which are shown in a drawing, in which:
Fig. 1 shows a diagrammatic longitudinal cross section of a respiration apparatus;
Fig. 2 shows a diagrammatic cross section of the respiration housing of the respiration apparatus of Fig. 1;
Fig. 3 shows a diagrammatic longitudinal cross section of a first embodiment of a valve housing;
Fig. 4 shows a diagrammatic longitudinal cross section of a second embodiment of a valve housing;
Fig. 5 shows a diagrammatic longitudinal cross section of a third embodiment of a valve housing; and
Fig. 6 and Fig. 7 each show a diagrammatic longitudinal cross section of a variant of the valve housing, in which the overpressure protection is settable.

The drawings are only diagrammatic views of preferred embodiments of the invention which are provided by way of non-limiting exemplary embodiments. In the drawings, same or corresponding parts are designated by the same reference numerals.

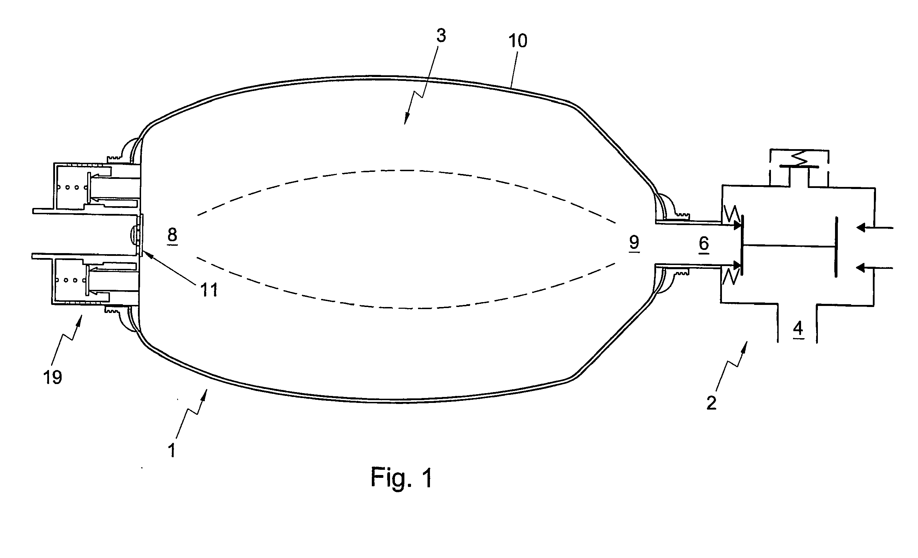

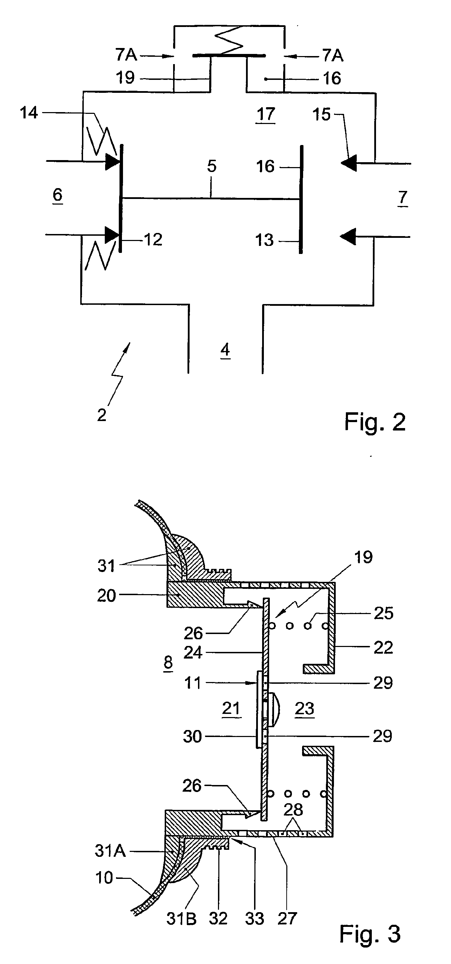

Fig. 1 shows a manually operated respiration apparatus 1, comprising a respiration housing 2 and a balloon unit 3 connected thereto. The respiration housing 2, which is shown in detail in Fig. 2, is provided with a respiration channel 4 which is connected via a valve system 5 with an inflow port 6 connected to the balloon unit 3 for supplying air to the patient via the respiration channel 4. The respiration channel 4 is further connected via the valve system 5 with one or more outflow ports 7 for evacuating the air exhaled by the patient into the respiration channel to the environment. The respiration channel 4 is, by its free end, connected with a mask (not shown) which is, during use, placed over the mouth and nose of the patient to whom artificial respiration is to be applied, or with a tube (not shown either) placed in the respiratory tract for artificial respiration.

The balloon unit 3 comprises a resilient bellows 10 provided with an inlet opening 8 and an outlet opening 9. The resilient bellows 10 may, for instance, be designed as an airtight wall from flexible material, for instance plastic, which is supported by resilient, arched ribs. The resilient bellows 10 may also be formed in a different manner, for instance as a hollow, somewhat elongated ball with relatively thick walls from rubberlike material. Such a balloon unit is known to a skilled person as a balloon unit of the self-priming type and will not be further elucidated here.

In the inlet opening 8 of the bellows 10, a non-return valve 11 is included in a manner which will be further elucidated hereinbelow. The outlet opening 9 of the bellows 10 is connected with the inflow port 6 of the respiration housing 2. Upon squeezing the bellows 10 from the starting position shown in Fig. 1, air enclosed by the bellows exits via the outlet opening 9. With reference to Fig. 2, the exiting air enters the inflow port 6 of the respiration housing 2 and flows via non-return valve 12 of the valve system 5 into the respiration channel 4. The valve body simultaneously closes off outflow ports 7. Via the respiration channel 4 and the mask, the air is supplied via nose and mouth to the lungs of the patient. Upon releasing the bellows 10, the bellows rebounds to the starting position shown in Fig. 1 by sucking in environmental air via the non-return valve 11 provided in the inlet opening 8. Thereby, the non-return valve 12 in the respiration housing 2 slams shut.

With reference to Fig. 2, during and after the rebound of the resilient bellows 10, the patients will exhale air into the respiration channel 4. By air exhaled into the respiration channel 4 and/or by action of spring 14, the valve body 13 of valve system 5 will get off its seating 15 to the position shown in Fig. 2 in which the exhaled air can be evacuated to the environment via the outflow ports 7 of the respiration housing 2.

The respiration housing 2 is provided with an overpressure protection for evacuating air to the environment when a predetermined pressure value in the respiration housing is exceeded. In an exemplary embodiment, the overpressure protection is designed as a spring-actuated valve 16 in the wall of a chamber 17 in the respiration housing 2, which chamber connects to the inflow port 6. Given an elevated pressure, the valve body of the overpressure protection 16 is pressed from its seating 19 against the action of the spring, so that the air can pass the wall of the chamber 17 and can be evacuated to the environment. Given an overpressure, the air can thus pass the overpressure protection 16 and be evacuated to the environment via outflow ports 7A. The construction of the respiration housing for a respiration apparatus is well enough known to a skilled person and will not be further elucidated here.

With reference to Fig. 1, in the inlet opening 8 of the bellows 10, an overpressure protection 19 is included for evacuating air from the bellows via the inlet opening 8 when a predetermined pressure value is exceeded. The overpressure protection included in the inlet opening comes into action when, as a result of squeezing the balloon too forcefully, the pressure in the balloon exceeds a predetermined value. By placement in the inlet opening 8, the pressure wave may be prevented from moving via the respiration housing 2 and the respiration channel 4 into the lungs of the patient.

With reference to Fig. 3, it is shown therein that, in the inlet opening 8 of the bellows 10, a substantially cylindrical valve housing 20 is included in which the non-return valve 11 and the overpressure protection 19 are integrated.

In the embodiment shown in Fig. 3, the valve housing 20 is provided with a central passageway 21 extending from a suction opening 23 provided in the end face 22 of the valve housing 20 to the interior of the bellows 10.

In the central passageway 21, a valve body 24 is included which is pressed against a seating 26 by action of a spring 25. The valve body 24, spring 25 and seating 26 together form the overpressure protection 19 which, during normal use, prevents outflow of gas from the balloon via the inlet opening 8. Given an overpressure caused by squeezing the bellows too forcefully, for instance given a pressure in the bellows of 60 cm water column, the valve body 24 will get off its seating 26 against the action of the spring 25, so that air from the bellows can flow out to the environment via outflow openings 28 provided in the jacket surface 27 of the valve housing 20, near the end face 22.

In the valve body 24, passage openings 29 are provided which are sealed on a side of the valve body 24 facing the bellows by means of a flexible valve body 30. The passage openings 29 and the flexible valve body 30 form a non-return valve 12 which prevents exiting of air from the bellows during squeezing, but which allows inflow of air from the environment to the interior of the bellows during the rebound.

The valve housing 20 is provided with clamping means 31 for airtight clamping of the circumferential edge of the inlet opening 8 of the bellows 10. In the embodiment shown in Fig. 3, the clamping means are formed by a supporting flange 31A provided on the valve housing 20, which cooperates with a pressure flange 31B of a screw ring 32 which is attached on the jacket 27 of the valve housing 20 by means of screw thread 33.

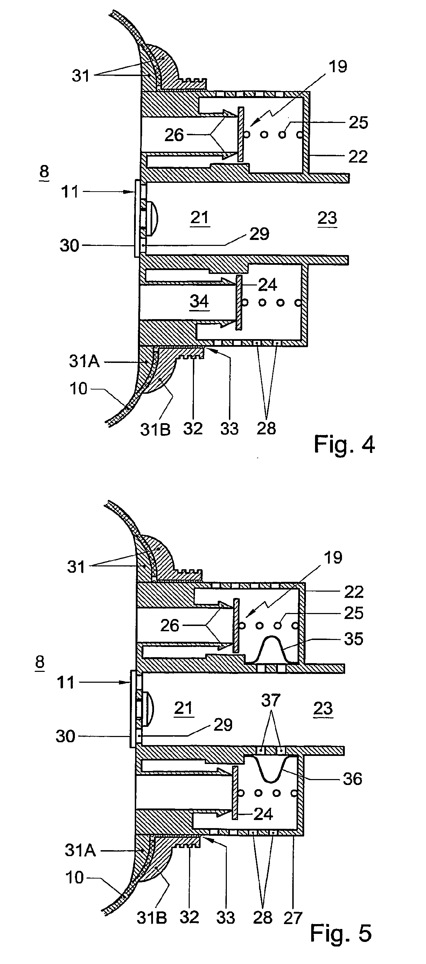

With reference to Fig. 4, a second embodiment of the valve housing 20 is shown therein, in which the valve body 24 has an annular design and is located in a blow-off channel 34 arranged around the central passageway 21. Here, the suction opening 23 has a cylindrical design and the blow-off channel 34 is arranged annularly around it. Here, the passageway 21 extends somewhat outwardly with respect to the end face 22, so that the suction opening 23 is located outside the end face 22. In this manner, the inflow opening can easily be connected to a bag. Via the bag, enriched air can be supplied to the balloon, for instance air with extra oxygen and/or a small amount of narcotic, such as laughing gas, for relief. Then, besides air, enrichment gas can likewise be continuously supplied to the bag from a gas cylinder with a relatively low flow rate, for instance 0-15 liters per minute.

Optionally, as shown in Fig. 5, an overpressure protection 35 may be provided in the wall of the central passageway 21 so that, when the pressure in the oxygen bag exceeds a predetermined value, oxygen can directly be evacuated to the blow-off channel 34. An example of such an overpressure protection is a duckbill valve 36 in the shape of a ring which clears passage openings 37 in the wall of the passageway given a pressure of approx 15 cm water column in the oxygen bag. For the record, it is noted that, when the respiration apparatus is coupled with an oxygen bag, the term air is to be interpreted to mean oxygen in this context.

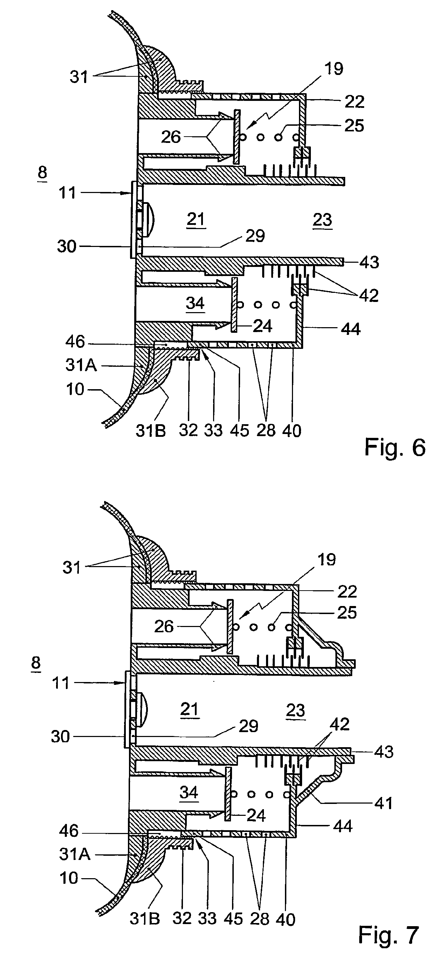

Fig. 6 shows a variant of the valve housing where the predetermined pressure value of the overpressure protection is settable. In this variant, the pressure value of the overpressure protection is settable between a pressure of approx 20 cm water column and a pressure of approx 60 cm water column. Of course, for less common applications of manual artificial respiration, an overpressure protection with a higher maximum pressure may be possible, for instance an overpressure protection with a setting range up to approx 80 or 120 cm water column.

Here, the settable overpressure protection is designed with a setting element designed as with a rotatable part 40 of the housing of the valve housing. The rotatable part 40 is connected with a stationary part 43 of the valve housing via a screw thread connection 42.

Via the screw thread connection 42, the rotatable part 40 of the valve housing 20 is rotatable over an angle range of approx 270° with respect to the stationary part 43 of the valve housing. For obtaining a simple, unambiguous operation, the angle range is less than approx 360°, and is preferably between approx 90° and 360°. In this manner, the whole setting range can be traversed with a pulse, while further, with each angular position, exactly one pressure value corresponds. For the purpose of a stable construction, the jacket 45 of the rotatable part 40 is included in an annular guide 46.

Preferably, further, the closing pressure exerted on the valve body 24 by means of spring 25 can be set linearly. By using a screw thread 42 with variable pitch, the spring 25 can have a non-linear design, while a linear setting characteristic is still obtained across the angle range.

In an advantageous manner, the end face 44 of the rotatable part 40 of the valve housing may be graduated for reading the set value of the overpressure protection on the basis of the relative angular position between the adjustable part of the valve housing 40 with respect to the stationary part 43. For this purpose, for instance an annular graduation may be represented on the part of the end face 44 surrounding the stationary part 43 of the valve housing, while a reference line is provided on the end face of the screw ring 32 and/or the end face of the stationary part 43. The settable overpressure protection may of course also be applied to other variants of the valve housing.

With reference to Fig. 7, it is shown that the rotatable part 40 of the housing of the valve housing is provided with a control button 41 to facilitate the engaging.

Optionally, the suction channel 23 surrounded by the stationary part 43 of the valve housing may also be provided with a protective cap with passage openings, for instance a protective grid.

With the aid of the settable overpressure protection, the air present in the bellows can thus be blown off via a settable barrier in or to the bellows. It will be clear that the settable overpressure protection may further be designed in many different manners than the preferred embodiment described hereinabove. For instance, a settable overpressure protection may be realized with the aid of a slidable stop or an adjustable clamp and the adjustment can be carried out either continuously or discontinuously. Further, if desired, the predetermined pressure value may be non-linearly settable across the setting range.

It will further be clear that such a settable overpressure protection in itself can already be advantageously applied in conventional manually operated respiration apparatuses, in other words, a respiration apparatus in which the overpressure protection is not provided in or to the balloon unit. Such a manually operated respiration apparatus then comprises a respiration housing, provided with a respiration channel for connection with a patient to whom artificial respiration is to be applied, which respiration channel is connected via a valve system with an inflow port connected to a balloon unit for supplying air to the patient via the respiration channel and with one or more outflow ports for evacuating air exhaled by the patient into the respiration channel to the environment, while the balloon unit comprises a resilient bellows provided with an inlet opening and an outlet opening, while, further, in the inlet opening of the bellows, a non-return valve is included and while the outlet opening of the bellows is connected with the inflow port of the respiration housing, and while the respiration apparatus is provided with an overpressure protection for evacuating air from the respiration apparatus when a predetermined pressure value is exceeded, with the predetermined pressure value of the overpressure protection being settable.

It will be clear that the invention is not limited to the preferred exemplary embodiments shown herein, but that many variations are possible. For instance, the overpressure protections may be designed as overpressure protections with a settable initial value. Further, the overpressure protection may be integrated in the inlet opening with the non-return valve, for instance by designing a flexible valve body of a non-return valve such that it will leak in case of overpressure. Such variants will be clear to a skilled person and are understood to be within the scope of the invention as set forth in the following claims.

## Claims

1. A manually operated respiration apparatus (1), comprising a respiration housing (2), provided with a respiration channel (4) for connection with a patient to whom artificial respiration is to be applied, which respiration channel (4) is connected via a valve system (5) with an inflow port (6) connected to a balloon unit (3) for supplying air to the patient via the respiration channel (4) and with one or more outflow port (7) for evacuating air exhaled by the patient into the respiration channel to the environment, wherein the balloon unit (3) comprises a resilient bellows (10) provided with an inlet opening (8) and an outlet opening (9), wherein, further, in the inlet opening (8) of the bellows (10), a non-return valve (11) is included and wherein the outlet opening (9) of the bellows (10) is connected with the inflow port (6) of the respiration housing (2), **characterized in that** the bellows (10) is provided with an overpressure protection (16) for evacuating air from the bellows (10) when a predetermined pressure value is exceeded.

2. A respiration apparatus (1) according to claim 1, wherein the overpressure protection (16) is provided in or to the inlet opening (8) of the bellows (10).

3. A respiration apparatus (1) according to claim 1 or 2, wherein, in the inlet opening (8) of the bellows (10), a substantially cylindrical valve housing (20) is included in which the non-return valve (11) and the overpressure protection (16) are accommodated.

4. A respiration apparatus (1) according to claim 3, wherein the valve housing (20) is, on an end face (22), provided with one or more suction openings (23) and wherein a part of the jacket surface (27) of the valve housing (20) located near the end face (22) is provided with one or more blow-off openings.

5. A respiration apparatus (1) according to claim 3 or 4 , wherein the valve housing (20) is provided with clamping mean (31) for airtight clamping of the circumferential edge of the inlet opening (8) of the bellows (10).

6. A balloon unit (3) for a manually operated respiration apparatus, comprising a resilient bellows (10) provided with an inlet opening (8) and an outlet opening (9), wherein, in the inlet opening (8) of the bellows (10), a non-return valve (11) is included, **characterized in that** the bellows (10) is provided with an overpressure protection (16) for evacuating air from the bellows (10) when a predetermined pressure value is exceeded.

7. A balloon unit (3) according to claim 6, wherein the overpressure protection (16) is provided in or to the inlet opening (8) of the bellows (10).

8. A balloon unit (3) according to claim 6 or 7, wherein, in the inlet opening (8) of the bellows (10), a substantially cylindrical valve housing (20) is included in which the non-return valve (11) and the overpressure protection (16) are accommodated.

9. A balloon unit (3) according to claim 8, wherein the valve housing (20) is, at an end face (22), provided with one or more suction openings (23) and wherein a part of the jacket surface (27) of the valve housing (20) located near the end face (22) is provided with one or more blow-off openings.

10. A balloon unit (3) according to any one of claims 8 or 9, wherein the valve housing (20) is provided with clamping means (31) for airtight clamping of the circumferential edge of the inlet opening (8) of the bellows (10).

11. A balloon unit (3) or respiration apparatus (1) according to any of claims 1-10, wherein the overpressure protection (16) comprises a valve.

12. A valve housing (20) for a respiration apparatus (1) or a balloon unit (3) according to any one of the preceding claims, comprising a substantially cylindrical valve housing (20) for inclusion in or to a bellows, in which valve housing (20) a non-return valve (11) and an overpressure protection (16) are accommodated.

13. A valve housing (20) according to claim 12, wherein the valve housing is, on an end face (22), provided with one or more suction openings (23) and wherein a part of the jacket surface (27) of the valve housing (20) located near the end face (22) is provided with one or more blow-off openings.

14. A valve housing (20) according to claim 12 or 13, wherein the valve housing (20) is, on its jacket, provided with clamping means (31) for airtight clamping of the circumferential edge of the inlet opening (8) of the bellows (10).

15. A respiration apparatus (1), balloon unit (3) or valve housing (20) according to any one of claims 1-14, wherein the predetermined pressure value of the overpressure protection (16) is settable.

16. A respiration apparatus (1), balloon unit (3) or valve housing (20) according to claim 15, wherein the pressure value of the overpressure protection (16) is settable over a range, preferably up to a pressure of approx or 80 or approx

17. A respiration apparatus (1), balloon unit (3) or valve housing (20) according to claim 15 or 16, wherein the settable overpressure protection (16) is designed with a rotatable setting element (40).

18. A respiration apparatus (1), balloon unit (3) or valve housing (20) according to claim 17, wherein, for the purpose of setting the overpressure protection (16), the setting element is rotatable over an angle range of less then approx 360°.

19. A respiration apparatus (1) according to any of claims 15-18, wherein a graduation is provided for reading the set pressure value of the overpressure protection (16) on the basis of the relative angular position between the adjustable part (40) of the valve housing and the stationary part (43) of the valve housing.

20. A respiration apparatus (1) balloon unit (3) or valve housing (20) according to any of claims 15-19, wherein the predetermined pressure value is linearly settable.

## Patentansprüche

1. Manuell betriebenes Beatmungsgerät (1) mit einem Beatmungsgehäuse (2), das mit einem Beatmungskanal (4) zur Verbindung mit einem künstlich zu beatmenden Patienten versehen ist, wobei der Beatmungskanal (4) über ein Ventilsystem (5) mit einem an eine Balloneinheit (3) angeschlossenen Einströmungs-Port (6) verbunden ist, um dem Patienten über den Beatmungskanal (4) Luft zuzuführen, und mit einem oder mehreren Ausströmungs-Ports (7) verbunden ist, um von dem Patienten in den Beatmungskanal ausgeatmete Luft in die Umgebung zu evakuieren, wobei die Balloneinheit (3) einen elastischen Balg (10) aufweist, der mit einer Einlassöffnung (8) und einer Auslassöffnung (9) versehen ist, wobei ferner in der Einlassöffnung (8) des Balgs (10) ein Rückschlagventil (11) enthalten ist und wobei die Auslassöffnung (9) des Balgs (10) mit dem Einströmungs-Port (6) des Beatmungsgehäuse (2) verbunden ist,
**dadurch gekennzeichnet, dass** der Balg (10) mit einem Überdruckschutz (16) versehen ist, um Luft aus dem Balg (10) zu evakuieren, wenn ein vorbestimmter Druckwert überschritten wird.

2. Beatmungsgerät (1) nach Anspruch 1, bei dem der Überdruckschutz (16) in oder an der Einlassöffnung (8) des Balgs (10) vorgesehen ist.

3. Beatmungsgerät (1) nach Anspruch 1 oder 2, bei dem in der Einlassöffnung (8) des Balgs (10) ein im Wesentlichen zylindrisches Ventilgehäuse (20) enthalten ist, in dem das Rückschlagventil (11) und der Überdruckschutz (16) untergebracht sind.

4. Beatmungsgerät (1) nach Anspruch 3, bei dem das Ventilgehäuse (20) an einer Endfläche (22) mit einer oder mehreren Saugöffnungen (23) versehen ist und bei dem ein nahe der Endfläche (22) gelegener Teil der Mantelfläche (27) des Ventilgehäuses (20) mit einer oder mehreren Ausblasöffnungen versehen ist.

5. Beatmungsgerät (1) nach Anspruch 3 oder 4, bei dem das Ventilgehäuse (20) mit Klemmvorrichtungen (31) zum luftdichten Anklemmen des Umfangsrands der Einlassöffnung (8) des Balgs (10) versehen ist.

6. Balloneinheit (3) für ein manuell betriebenes Beatmungsgerät, mit einem elastischen Balg (10), der mit einer Einlassöffnung (8) und einer Auslassöffnung (9) versehen ist, wobei in der Einlassöffnung (8) des Balgs (10) ein Rückschlagventil (11) enthalten ist, **dadurch gekennzeichnet, dass** der Balg (10) mit einem Überdruckschutz (16) versehen ist, um Luft aus dem Balg (10) zu evakuieren, wenn ein vorbestimmter Druckwert überschritten wird.

7. Balloneinheit (3) nach Anspruch 6, bei dem der Überdruckschutz (16) in oder an der Einlassöffnung (8) des Balgs (10) vorgesehen ist.

8. Balloneinheit (3) nach Anspruch 6 oder 7, bei dem in der Einlassöffnung (8) des Balgs (10) ein im Wesentlichen zylindrisches Ventilgehäuse (20) enthalten ist, in dem das Rückschlagventil (11) und der Überdruckschutz (16) untergebracht sind.

9. Balloneinheit (3) nach Anspruch 8, bei dem das Ventilgehäuse (20) an einer Endfläche (22) mit einer oder mehreren Saugöffnungen (23) versehen ist und bei dem ein nahe der Endfläche (22) gelegener Teil der Mantelfläche (27) des Ventilgehäuses (20) mit einer oder mehreren Ausblasöffnungen versehen ist.

10. Balloneinheit (3) nach einem der Ansprüche 8 oder 9, bei dem das Ventilgehäuse (20) mit Klemmvorrichtungen (31) zum luftdichten Anklemmen des Umfangsrands der Einlassöffnung (8) des Balgs (10) versehen ist.

11. Balloneinheit (3) oder Beatmungsgerät (1) nach einem der Ansprüche 1 - 10, bei der bzw. dem der der Überdruckschutz (16) ein Ventil aufweist.

12. Ventilgehäuse (20) für ein Beatmungsgerät (1) oder eine Balloneinheit (3) nach einem der vorgehenden Ansprüche, das ein im Wesentlichen zylindrisches Ventilgehäuse (20) zur Anordnung in oder an einem Balg aufweist, wobei in dem Ventilgehäuse (20) ein Rückschlagventil (11) und ein Überdruckschutz (16) untergebracht sind.

13. Ventilgehäuse (20) nach Anspruch 12, wobei das Ventilgehäuse an einer Endfläche (22) mit einer oder mehreren Saugöffnungen (23) versehen ist und bei dem ein nahe der Endfläche (22) gelegener Teil der Mantelfläche (27) des Ventilgehäuses (20) mit einer oder mehreren Ausblasöffnungen versehen ist.

14. Ventilgehäuse (20) nach Anspruch 12 oder 13, wobei das Ventilgehäuse an seiner Mantelfläche mit Klemmvorrichtungen (31) zum luftdichten Anklemmen des Umfangsrands der Einlassöffnung (8) des Balgs (10) versehen ist.

15. Beatmungsgerät (1), Balloneinheit (3) oder Ventilgehäuse (20) nach einem der Ansprüche 1-14, bei dem bzw. der der vorbestimmte Druckwert des Überdruckschutzes (16) einstellbar ist.

16. Beatmungsgerät (1), Balloneinheit (3) oder Ventilgehäuse (20) nach Anspruch 15, bei dem bzw. der der Druckwert des Überdruckschutzes (16) über einen Bereich einstellbar ist, vorzugsweise bis zu einem Druck von ungefähr 80 oder ungefähr 120 cm Wassersäule.

17. Beatmungsgerät (1), Balloneinheit (3) oder Ventilgehäuse (20) nach Anspruch 15 oder 16, bei dem bzw. der der einstellbare Überdruckschutz (16) mit einem drehbaren Einstellelement (40) versehen ist.

18. Beatmungsgerät (1), Balloneinheit (3) oder Ventilgehäuse (20) nach Anspruch 17, bei dem bzw. der zwecks Einstellens des Überdruckschutzes (16) das Einstellelement über einen Winkelbereich von weniger als ungefähr 360° drehbar ist.

19. Beatmungsgerät (1) nach einem der Ansprüche 15-18, bei dem eine Gradeinteilung vorgesehen ist, um den eingestellten Druckwert des Überdruckschutzes (16) auf der Basis der relativen Winkelposition zwischen dem einstellbaren Teil (40) des Ventilgehäuses und dem stationären Teil des (43) Ventilgehäuses zu lesen.

20. Beatmungsgerät (1), Balloneinheit (3) oder Ventilgehäuse (20) nach einem der Ansprüche 15-19, bei dem bzw. der der vorbestimmte Druckwert linear einstellbar ist.

## Revendications

1. Appareil de respiration à commande manuelle (1) comprenant un logement de respiration (2), pourvu d'un canal de respiration (4) pour une liaison à un patient auquel une respiration artificielle doit être appliquée, lequel canal de respiration (4) est relié, par l'intermédiaire d'un système de valve (5), à un orifice d'entrée (6) relié à une unité de ballon (3) pour fournir de l'air au patient par l'intermédiaire du canal de respiration (4) et à un ou plusieurs orifices de sortie (7) pour évacuer l'air exhalé par le patient dans le canal de respiration dans l'environnement, dans lequel l'unité de ballon (3) comprend un soufflet (10) élastique pourvu d'une ouverture d'entrée (8) et d'une ouverture de sortie (9), dans lequel, en outre, dans l'ouverture d'entrée (8) du soufflet (10), un clapet de non-retour (11) est inclus, et dans lequel l'ouverture de sortie (9) du soufflet (10) est reliée à l'orifice d'entrée (6) du logement de respiration (2), **caractérisé en ce que** le soufflet (10) est pourvu d'une protection contre une surpression (16) pour évacuer l'air du soufflet (10) lorsqu'une valeur de pression prédéterminée est dépassée.

2. Appareil de respiration (1) selon la revendication 1, dans lequel la protection contre une surpression (16) est prévue dans ou sur l'ouverture d'entrée (8) du soufflet (10).

3. Appareil de respiration (1) selon la revendication 1 ou 2, dans lequel, dans l'ouverture d'entrée (8) du soufflet (10), un logement de valves (20) sensiblement cylindrique est inclus, dans lequel le clapet de non-retour (11) et la protection contre une surpression (16) sont logés.

4. Appareil de respiration (1) selon la revendication 3, dans lequel le logement de valves (20) est, sur une face d'extrémité (22), pourvu d'une ou de plusieurs ouvertures d'aspiration (23), et dans lequel une partie de la surface de chemise (27) du logement de valves (20) située à proximité de la face d'extrémité (22) est pourvue d'une ou de plusieurs ouvertures d'évacuation.

5. Appareil de respiration (1) selon la revendication 3 ou 4, dans lequel le logement de valves (20) est pourvu de moyens de serrage (31) pour le serrage hermétique du bord circonférentiel de l'ouverture d'entrée (8) du soufflet (10).

6. Unité de ballon (3) destinée à un appareil de respiration à commande manuelle, comprenant un soufflet (10) élastique pourvu d'une ouverture d'entrée (8) et d'une ouverture de sortie (9), dans laquelle, dans l'ouverture d'entrée (8) du soufflet (10), un clapet de non-retour (11) est inclus, **caractérisée en ce que** le soufflet (10) est pourvu d'une protection contre une surpression (16) pour évacuer l'air du soufflet (10) lorsqu'une valeur de pression prédéterminée est dépassée.

7. Unité de ballon (3) selon la revendication 6, dans laquelle la protection contre une surpression (16) est prévue dans ou sur l'ouverture d'entrée (8) du soufflet (10).

8. Unité de ballon (3) selon la revendication 6 ou 7, dans laquelle, dans l'ouverture d'entrée (8) du soufflet (10), un logement de valves (20) sensiblement cylindrique est inclus, dans lequel le clapet de non-retour (11) et la protection contre une surpression (16) sont logés.

9. Unité de ballon (3) selon la revendication 8, dans laquelle le logement de valves (20) est, sur une face d'extrémité (22), pourvu d'une ou de plusieurs ouvertures d'aspiration (23), et dans laquelle une partie de la surface de chemise (27) du logement de valves (20) située à proximité de la face d'extrémité (22) est pourvue d'une ou de plusieurs ouvertures d'évacuation.

10. Unité de ballon (3) selon l'une quelconque des revendications 8 ou 9, dans laquelle le logement de valves (20) est pourvu de moyens de serrage (31) pour le serrage hermétique du bord circonférentiel de l'ouverture d'entrée (8) du soufflet (10).

11. Unité de ballon (3) ou appareil de respiration (1) selon l'une quelconque des revendications 1 à 10, dans lequel la protection contre une surpression (16) comprend une valve.

12. Logement de valves (20) destiné à un appareil de respiration (1) ou une unité de ballon (3) selon l'une quelconque des revendications précédentes, comprenant un logement de valves (20) sensiblement cylindrique destiné à être inclus dans ou sur un soufflet, dans lequel logement de valves (20), un clapet de non-retour (11) et une protection contre une surpression (16) sont logés.

13. Logement de valves (20) selon la revendication 12, dans lequel le logement de valves est, sur une face d'extrémité (22), pourvu d'une ou de plusieurs ouvertures d'aspiration (23), et dans lequel une partie de la surface de chemise (27) du logement de valves (20) située à proximité de la face d'extrémité (22) est pourvue d'une ou de plusieurs ouvertures d'évacuation.

14. Logement de valves (20) selon la revendication 12 ou 13, dans lequel le logement de valves (20) est, sur sa chemise, pourvu de moyens de serrage (31) pour le serrage hermétique du bord circonférentiel de l'ouverture d'entrée (8) du soufflet (10).

15. Appareil de respiration (1), unité de ballon (3) ou logement de valves (20) selon l'une quelconque des revendications 1 à 14, dans lequel la valeur de pression prédéterminée de la protection contre une surpression (16) peut être réglée.

16. Appareil de respiration (1), unité de ballon (3) ou logement de valves (20) selon la revendication 15, dans lequel la valeur de pression de la protection contre une surpression (16) peut être réglée dans une plage, de préférence jusqu'à une pression d'approximativement 80 ou d'approximativement 120 cm de colonne d'eau.

17. Appareil de respiration (1), unité de ballon (3) ou logement de valves (20) selon la revendication 15 ou 16, dans lequel la protection contre une surpression (16) réglable est conçue avec un élément de réglage rotatif (40).

18. Appareil de respiration (1), unité de ballon (3) ou logement de valves (20) selon la revendication 17, dans lequel, afin de régler la protection contre une surpression (16), l'élément de réglage est capable de tourner dans une plage angulaire inférieure à approximativement 360°.

19. Appareil de respiration (1) selon l'une quelconque des revendications 15 à 18, dans lequel une graduation est prévue pour lire la valeur de pression réglée de la protection contre une surpression (16) sur la base de la position angulaire relative entre la partie ajustable (40) du logement de valves et la partie fixe (43) du logement de valves.

20. Appareil de respiration (1), unité de ballon (3) ou logement de valves (20) selon l'une quelconque des revendications 15 à 19, dans lequel la valeur de pression prédéterminée peut être réglée linéairement.
